# EUROPEAN PATENT APPLICATION

(11) **EP 4 768 022 A1**
(43) Date of publication of application: **01.07.2026**
(21) Application number: 25209724.1
(22) Date of filing: 20.10.2025
(51) Int. Cl.: A61K 9/48, A61K 31/165

(54) **A CAPSULE FORMULATION OF LISDEXAMFETAMINE DIMESYLATE**

(30) Priority: 24.12.2024 TR 2024020286
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); BENGUL, Furkan, Istanbul (TR)
(74) Representative: Genç Ilhan, Oznur

(57) **Abstract**

The present invention relates to a capsule formulation comprising lisdexamfetamine dimesylate and at least one filler, wherein filler is calcium phosphate dibasic anhydrous. Furthermore, the invention discloses a process which is simple, rapid, cost effective, timesaving and industrially convenient process and is created to eliminate the disadvantages of active ingredient.

## Description

### Field of the Invention

The present invention relates to a capsule formulation comprising lisdexamfetamine dimesylate and at least one filler, wherein filler is calcium phosphate dibasic anhydrous. Furthermore, the invention discloses a process which is simple, rapid, cost effective, time-saving and industrially convenient process and is created to eliminate the disadvantages of active ingredient.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate also referred to as I-lysine-d-amphetamine dimesylate of Formula I is chemically named as (2S)-2,6-Diamino-N-[(1S)-1-methyl-2-phenylethyl]hexanamide dimesylate.

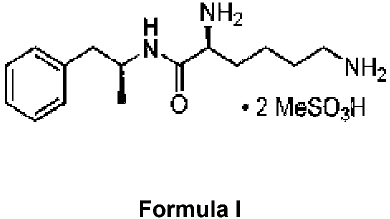

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50 and 60 mg hard capsules or chewable tablets. Each capsule or chewable tablet contains 10, 20, 30, 40, 50 or 60 mg lisdexamfetamine dimesylate, equivalent to 5.8 mg, 11.6 mg, 17.3 mg, 23.1 mg or 28.9 mg lisdexamfetamine, respectively.

The patent WO2006121552A2 discloses a capsule formulation containing lisdexamfetamine dimesylate, microcrystalline cellulose, croscarmellose sodium, magnesium stearate.

In the prior art formulation, microcrystalline cellulose was used as a filler. Microcrystalline cellulose is generally a safe compound widely used in pharmaceutical formulations. However, although microcrystalline cellulose is a good filler, it is known to have some disadvantages. Lisdexamfetamine Dimesylate is the deliquescent active substance and microcrystalline cellulose is a hygroscopic excipient. Because of its hygroscopic nature, Microcrystalline Cellulose is generally not an effective compound especially for those hygroscopic or deliquescent active substances. For the purpose of improving the stability of some active substances, such as lisdexamfetamine dimesylate, the hygroscopicity of microcrystalline cellulose can be problematic.

Therefore, there is still a need for a lisdexamfetamine dimesylate formulation with high stability and additional advantages over the prior art.

### Detailed Description of the Invention

The main object of the present invention is to provide a capsule formulation comprising lisdexamfetamine dimesylate having the high stability and flowability.

Another object of the present invention is to provide a process for a capsule formulation comprising lisdexamfetamine dimesylate prepared by simple, easy, time-saving and fast manufacturing methods.

Lisdexamfetamine dimesylate formulation is known in the prior art, but there is still a need for a formulation with high stability and flowability. Calcium phosphate dibasic anhydrous was used as filler in the formulation due to the moisture sensitivity of lisdexamfetamine dimesylate. The calcium phosphate dibasic anhydrous is a non-hygroscopic excipient. The calcium phosphate dibasic anhydrous used as a filler helps to optimize moisture absorption capacity of pharmaceutical product and improve powder flow in manufacturing. We found that the use of anhydrous calcium phosphate dibasic as filler in the formulation increased the stability of the formulation and improved the flowability.

According to one embodiment of the invention, a capsule formulation comprising lisdexamfetamine dimesylate and at least one filler, wherein filler is calcium phosphate dibasic anhydrous.

According to one embodiment of the present invention, the amount of the lisdexamfetamine dimesylate is between 25.0% and 45.0% by weight of the total formulation. Preferably, it is between 33.0% and 42.0% by weight of the total formulation.

According to this embodiment of the present invention, the amount of filler is between 45.0% and 65.0% by weight of the total formulation. Preferably, it is between 52.0% and 60.0% by weight of the total formulation.

According to one embodiment of the present invention, the capsule formulation further comprises at least one pharmaceutically acceptable excipient selected from the group comprising disintegrants, lubricants or mixtures thereof.

Suitable disintegrants are selected from the group comprising sodium starch glycolate, croscarmellose sodium, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is sodium starch glycolate.

According to one embodiment of the present invention, the amount of disintegrant is between 0.5% and 10.0% by weight of the total formulation. Preferably, it is between 2.0% and 6.0% by weight of the total formulation.

Suitable lubricants are selected from the group comprising sodium stearyl fumarate, magnesium stearate, talc, colloidal silicon dioxide, corn starch, calcium stearate, zinc stearate, magnesium lauryl sulfate, sodium oleate, sodium acetate, stearic acid, fumaric acid, glyceryl palmito sulphate or mixtures thereof.

According to one embodiment of the present invention, the lubricant is sodium stearyl fumarate.

According to one embodiment of the present invention, the amount of lubricant is between 0.1% and 5.0% by weight in the total formulation.

According to one embodiment of the present invention, the capsule formulation comprises;
- Lisdexamfetamine dimesylate
- Calcium phosphate dibasic anhydrous
- Sodium starch glycolate
- Sodium stearyl fumarate

According to this embodiment of the invention, a process for capsule formulations comprising lisdexamfetamine dimesylate comprises the following steps:
a. Sieving and blending lisdexamfetamine dimesylate and filler,
b. Sieving disintegrant and blending it with the powder mixture obtained in (a),
c. Sieving the lubricant and blending it with the powder mixture obtained in (b),
d. Filling the final mixture obtained in (c) into capsules.

### Example 1;

| **Ingredients** | **%by weight** | **%by weight** |
|---|---|---|
| Lisdexamfetamine dimesylate | 38.9 | 25-45 |
| Calcium phosphate dibasic anhydrous | 55.6 | 45-65 |
| Sodium starch glycolate | 3.9 | 0.5-10 |
| Sodium stearyl fumarate | 1.6 | 0.1-5 |
| **TOTAL** | **100** | **100** |
| **Gelatine Capsule** | | |

a. Sieving and blending lisdexamfetamine dimesylate and calcium phosphate dibasic anhydrous
b. Sieving Sodium starch glycolate and blending it with the powder mixture obtained in (a),
c. Sieving Sodium stearyl fumarate and blending it with the powder mixture obtained in (b),
d. Final mixture obtained in (c) is filled in capsules

## Claims

1. A capsule formulation comprising lisdexamfetamine dimesylate and at least one filler, wherein filler is calcium phosphate dibasic anhydrous.

2. The capsule formulation according to claim 1, wherein the amount of the lisdexamfetamine dimesylate is between 25.0% and 45.0% by weight of the total formulation.

3. The capsule formulation according to claim 1, wherein the amount of filler is between 45.0% and 65.0% by weight of the total formulation.

4. The capsule formulation according to claim 1, wherein the capsule formulation further comprises at least one pharmaceutically acceptable excipient selected from the group comprising disintegrants, lubricants or mixtures thereof.

5. The capsule formulation according to claim 4, wherein disintegrants are selected from the group comprising sodium starch glycolate, croscarmellose sodium, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

6. The capsule formulation according to claim 5, wherein the disintegrant is sodium starch glycolate.

7. The capsule formulation according to claim 5 or 6, wherein the amount of disintegrant is between 0.5% and 10.0% by weight of the total formulation.

8. The capsule formulation according to claim 4, wherein lubricants are selected from the group comprising sodium stearyl fumarate, magnesium stearate, talc, colloidal silicon dioxide, corn starch, calcium stearate, zinc stearate, magnesium lauryl sulfate, sodium oleate, sodium acetate, stearic acid, fumaric acid, glyceryl palmito sulphate or mixtures thereof.

9. The capsule formulation according to claim 8, wherein the lubricant is sodium stearyl fumarate.

10. The capsule formulation according to claim 8 or 9, wherein the amount of lubricant is between 0.1% and 5.0% by weight in the total formulation.

11. The capsule formulation according to any preceding claims, wherein the capsule formulation comprises;
- Lisdexamfetamine dimesylate
- Calcium phosphate dibasic anhydrous
- Sodium starch glycolate
- Sodium stearyl fumarate

12. A process for capsule formulation according to any of the preceding claims comprising lisdexamfetamine dimesylate comprising the following steps:
a. Sieving and blending lisdexamfetamine dimesylate and filler,
b. Sieving disintegrant and blending it with the powder mixture obtained in (a),
c. Sieving the lubricant and blending it with the powder mixture obtained in (b),
d. Filling the final mixture obtained in (c) into capsules.
